# EUROPÄISCHE PATENTANMELDUNG

(11) **EP 1 024 199 A2**
(43) Veröffentlichungstag der Anmeldung: **02.08.2000**
(21) Anmeldenummer: 00100980.2
(22) Anmeldetag: 19.01.2000
(51) Int. Cl.: C12P 7/64, C12N 1/10, A61K 31/202, A61K 7/00, A61K 7/48, A23K 1/16, A23L 1/30

(54) **Gewinnung von Gamma-Linolensäure aus Protozoen der Gattung Colpidium**

(30) Priorität: 27.01.1999 DE 19903095
(71) Anmelder: Axiva GmbH, 65926 Frankfurt am Main (DE)
(72) Erfinder: Kiy, Thomas, Dr., 65929 Frankfurt (DE); Brinkmann, Kay, 81476 München (DE)

(57) **Zusammenfassung**

Die vorliegende Erfindung betrifft ein Verfahren zur Herstellung von γ- Linolensäure (GLA), aus Colpidium und dessen Verwendung in Lebensmitteln, Kosmetika und Pharmaka. Colpidium läßt sich in einem optimierten Medium fermentativ bis zu einer hohen absoluten Ausbeute an GLA kultivieren.

## Beschreibung

Die Erfindung betrifft ein Verfahren zur Herstellung hochungesättigter Fettsäuren, vorzugsweise der physiologisch bedeutenden γ-Linolensäure (all-cis 6,9,12-Octadecatriensäure; 18:3 n-6; nachfolgend: "GLA") aus Protozoen der Gattung Colpidium und deren Verwendung.

Das Interesse der Industrie an der Gewinnung und Isolierung von Fettsäuren, insbesondere von ernährungsphysiologisch wichtigen (essentiellen) Fettsäuren, vorzugsweise mehrfach ungesättigte Fettsäuren, ist groß. Insbesondere gilt die Erschließung und Auswahl neuer biologischer Quellen, welche kostengünstig GLA liefern könnten, eine besondere Aufmerksamkeit. GLA wird in der Hauptsache ressourcenabhängig aus pflanzlichen Ölen gewonnen.

Insbesondere hochungesättigte Fettsäuren (sogenannte "PUFA" für: polyunsaturated fatty acids) sind von wirtschaftlicher Bedeutung, da sie (1.) als Lebensmittelzusatzstoffe (in Babynahrung u.v.a; siehe WO 91/11918), (2.) als pharmazeutische Wirkstoffe in einer Vielzahl von Indikationen und (3.) als Bestandteile von Kosmetika positive Effekte aufweisen.

Im humanen und tierischen Stoffwechsel ist die Delta-6 Desaturase das Schlüsselenzym zur Synthese der γ-Linolensäure aus der Linolsäure (18:2 n-6). Von der GLA leiten sich alle anderen ω-6 PUFAs und einige Eicosanoid-Hormone ab. Bei einer zu niedrigen Aktivität der Delta-6 Desaturase - verursacht beispielsweise durch Alter, Mangelernährung oder Alkoholismus - kommt es zu einer Unterversorgung an GLA und ihrer Folgeprodukte, wodurch eine Reihe von Erkrankungen verursacht werden können.

Daher dient GLA zur humanen und veterinären Behandlung von Entzündungs- und Immunkrankheiten (Wu, D., Meydani, 5. N., 1996; γ-Linolenic Acid, Metabolism and its role in nutrition and medicine (ed. Huang and Mills), 1996 AOCS, 106-117), bei Herz-Kreislauferkrankungen (Deferne, J.L. & Leeds, 1992; J. Hum. Hypertension, 6, 113-119), insbesondere Bluthochdruck, Diabetes (Horrobin, D.F.,1988; Prog. Lipid Res. 31,163-194) und bestimmten Krebsformen (Das, U.N, 1996; γ-Linolenic Acid, Metabolism and its role in nutrition and medicine (ed. Huang and Mills),1996 AOCS, 106-117). Bekannt ist ebenfalls der Einsatz von GLA zur Prophylaxe und zur Behandlung von chronischen, degenerativen Krankheiten, insbesondere rheumatischer Arthritis.

Säugetiere wandeln GLA in Dihomo-GLA (20:3 n-6; DGLA) um und reichem DGLA in der Muttermilch an. In humaner Muttermilch variiert der Gehalt an GLA von 0,35 bis 1,0% (Gibson, R.A., Kneebone, 1981; Am. J. Clin. Nutr., 34, 252-257). GLA findet daher Verwendung in der Nahrungsmittelindustrie, insbesondere in der Säuglingsemährung.

Die Bereitstellung nativer GLA-Vorkommen erfolgt vor allem in höheren Pflanzen und in einer Reihe von Mikroorganismen (Phillips, J.C., Huang, Y.-S., 1996; γ-Linolenic Acid, Metabolism and its role in nutrition and medicine (ed. Huang and Mills),1996 AOCS, 106-117), wie:

Familie: Onagraceae: Die Samen der Nachtkerze (Oenothera biennis) enthalten bis zu 24% Öl (Whipkey, A., Simon, J.E., Janick, J., 1988; J. Am. Oil Chem. Soc. 65, 979-984); dieses enthält 7-14% GLA (Wolf, R.B., Kleiman, R., England, R.E., 1983, J. Am. Oil Chem. Soc. 60, 1858-1860). Das Nachtkerzen-Öl ist die am häufigsten benutzte GLA-Quelle für klinische und pharmazeutische Anwendungen (Horrobin, D.F.1992; Prog. Lipid Res. 31, 163-194).

Familie: Boraginaceae: Die Samenöle von Borago officinalis (Boretsch) und Symphytum officinale enthalten einen GLA-Anteil von 20-27% (Kleiman, R., Earle, F.R., Wolff, I.A., Jones, Q. 1964; J.Am. Oil Chem. Soc. 41, 209-11). Das Boretsch-Öl weist jedoch höhere Mengen an längerkettigen, einfach ungesättigten Fettsäuren auf und enthält toxische ungesättigte Pyrrolidizin-Alkaloide.

Familie: Saxifragaceae: Die Samen der Schwarzen Johannisbeere (Ribes nigrum) enthalten bis zu 19% GLA (Traitler, H., Wille, H.J., Studer, A., 1988; J. Am. Oil Chem. Soc. 65, 755-760).

Bekannt sind ebenfalls GLA - haltige Öle aus Fermentation von Mikroorganismen, wie:

Pilze der Gattungen
- Mortierella (M. ramanniana, GLA-Gehalt des extrahierbaren Öls ca. 25%) (Hansson, L., Dostalek, M., 1988; Appl. Microbiol. Biotechnol.: 28, 240-6)
- Mucor (M. rouxii, und M. alpina, GLA-Gehalt des extrahierbaren Öls ca. 17%) (Lindberg, A.M., Hansson, L., 1991; Appl. Microbiol. Biotechnol., 36, 26-8; Shimitzu, S., Shinmen, Y., Kawashima, H., Akimoto, K., Yamada, H., 1988; Proceedings of ISF-JOCS World Congress 1988, 1000-6);
- Phycomyceten (P. blakesleeanus, GLA-Gehalt des extrahierbaren Öls ca. 16%) (Shaw, R., 1965; Biochim. Biophys. Acta, 98, 230-7);
- Rhizopus arrhizus (Kristofikova, L., Rosenberg, M., Vlnova, A., Sajbidor, J., Certik, M., 1991; Folia Microbiol., 36, 451-5)

"Algen" der Gattung
- Spirulina (5. platensis, GLA-Gehalt des extrahierbaren Öls 12 - 26%) (Mahajan G., Kamat, M., 1995; Appl. Microbiol. Biotechnol., 43, 466-9; Nichols, B.W., Wood, B.J.B., 1968; Lipids, 3, 46-50),

sowie Protozoen der Gattung
- Tetrahymena rostrata: GLA-Gehalt des extrahierbaren Öls ca. 21%; (nach: Gosselin, Y., Lognay, G., Thonart, P., 1989; Biotechnol. Lett., 11 (6), 423-6) Tetrahymena thermophila, GLA-Gehalt des extrahierbaren Öls ca. 33% (nach Kiy, T., 1993; Dissertation).

Gegenüber den anderen genannten biologischen Organismen sind die Protozoen als Quellen zur Gewinnung von hochungesättigten Fettsäuren wenig beschrieben und für die technische GLA-Gewinnung weitgehendst unerschlossen.

Ihre Vorteile gegenüber den anderen bekannten biologischen Quellen bestehen
(1.) in einer Vielfalt jeweils charakteristischer Fettsäure-Spektren mit z.T. einer überwiegenden Hauptkomponente im Öl;
(2.) möglicher Kultivierbarkeit in einem Bioreaktor, der Fermentation;
(3.) einer exakt zu kontrollierenden Fermentation, d.h. von Umwelteinflüssen unabhängigen Kultivierung;
(4.) und einem an die Fermentation anschließenden definierten Aufarbeitungsprozeß;
(5.) sind die Generationszeiten bedeutend kürzer als bei Pflanzen und Pilzen, so daß sich höhere Raum-Zeit-Ausbeuten in der Produktion ergeben.

Fermentation von Bakterien, Cyanobakterien, Algen, Pilzen und Zellkulturen aus vielzelligen Geweben pflanzlichen oder tierischen Ursprungs sind im Stand der Technik beschrieben, ihre Anreicherung, Aufarbeitung, Reinigung z.B. innerhalb einer Fermentation sind jedoch auf Protozoen nicht übertragbar.

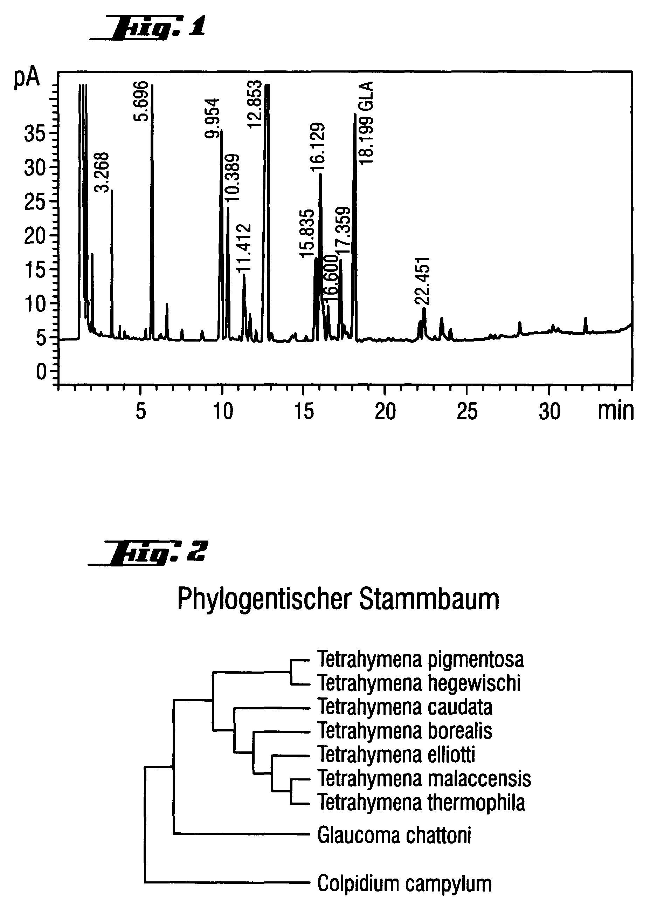

Fermentationsbedingungen für Protozoen (Kiy, Protist, 149, 1998) werden erst seit jüngster Zeit entwickelt. So ist die Fermentation von Tetrahymena-Arten beschrieben, welche jedoch nicht übertragbar ist auf andere verwandte Arten innerhalb der Protozoen. Nachteilig ist zudem das breite Fettsäurespektrum mit mehreren ungesättigten Fettsäuren, welche technisch aufwendig zu trennen sind (vgl. Fig. 1) und die Gewinnung von GLA nicht spezifiziert. In anderen Protozoen, wie Paramecium caudatum und Colpoda steini (Dembitskii, V.M., Zharikova, N.I. Inst. Zool. Tolyatti, USSR. Khim. Prir. Soedin. (1998) 2, 294-5) konnten nur Spuren von GLA nachgewiesen werden.

Bei gewünschter großtechnischer Produktion beeinträchtigen daher die Begleitstoffe der gewählten biologischen Quelle die Qualität der erhaltenen GLA - haltigen Biomasse. Dies kann eine Beeinträchtigung der genannten prophylaktischen und medizinischen Wirkung bewirken und erfordert daher eine komplexe Anreicherung und Aufreinigung. Zudem soll die zu gewinnende GLA als Nahrungsmittel und Arzneimittel eingesetzt werden, hierzu bedarf es einer biologischen Quelle, welche nicht-pathogen, insbesondere nicht-humanpathogen, ist.

Des weiteren ist es vonnöten, eine wirtschaftliche, kostengünstige Gewinnung samt Aufreinigung zu etablieren, die eine industrielle Nutzung ermöglicht.

Aufgabe der vorliegenden Erfindung ist daher ein Verfahren zur Herstellung von GLA aus Protozoen, in reinster möglichst angereicherter Form.

Die Aufgabe wird überraschender Weise durch die spezifische Auswahl des Protozoen aus der Gattung Colpidium gelöst, besonders bevorzugt ist die Art Colpidium campylum. Colpidium weist einen hohen GLA-Gehalt im Fettsäurespektrum auf (Vgl. Fig. 1). Die GLA - Fraktion ist frei von Verunreinigung in Form anderer in ihren Eigenschaften der GLA ähnlicher Fettsäuren (wie ALA), wodurch die Aufreinigung der GLA unmittelbar erleichtert wird und GLA aus der Biomasse in reiner Form kostengünstig erhältlich ist. Dies ist besonders dann beachtlich, falls die technische Gewinnung dieser ungesättigten Fettsäure mit Hilfe eines chromatographischen Verfahren erfolgt.

Protozoen im Sinne dieser Erfindung sind solche, wie folgt systematisiert (nach Cavalier Smith, T. (1995), Cell cycles diplokaryosis and the archezoan origin of sex;arch. Protistenk., 145 (3-4) 189-207): "Kingdom Protozoa" mit den Familien:
- 1.: Percolozoa
- 2.: Parabasalia
- 3.: Euglenozoa
- 4.: Mycetozoa
- 5.: Entamoeba
- 6.: Opalozoa
- 7.: Dinozoa
- 8.: Apicomplexa
- 9.: Ciliophora
- 10.: Haplosporidia
- 11.: Paramyxia
- 12.: Rhizopoda
- 13.: Reticulosa
- 14.: Heliozoa
- 15.: Radiozoa
- 16.: Amoebozoa
- 17.: Choanozoa

Innerhalb der Protozoen ist erfindungsgemäß bevorzugt aus Ciliophora (auch: "Ciliaten") die Gattung Colpidium, insbesondere Colpidium campylum, da Colpidium nicht human-pathogen ist. Daher ist ein Gegenstand der Erfindung die Verwendung der Gattung Colpidium als biologische Quelle zur Gewinnung und Isolierung von GLA.

Insbesondere weist Colpidium vorteilhaft für Protozoen große Zelldurchmesser von 50 µm (vgl. Tetrahymena: ca. 25 µm) auf, die in der präparativen Biomassen-Gewinnung, insbesondere in der Kultivierung und Fermentation, gezielt für höhere Raum-Zeit-Ausbeuten genutzt werden können. Zudem liegt das Optimum der Fermentation bei vorzugsweise 19 - 28 °C, besonders bevorzugt sind 22 - 25°C (vgl. Tetrahymena: ca. 30°C (Kiy, Tiedtke, Appl. Microbiol. Biotechnol, 37 (1992)).

Colpidium weist zu der als GLA-Produzent identifizierten Gattung Tetrahymena eine signifikante phylogenetische Distanz auf (Fig. 2).

Die erste Kultivierung der Protozoe Colpidium campylum ist durch Rogerson und Berger (Rogerson, A., Berger, J., 1981; J. Gen. Microbiol., 124, 53-9; Rogerson, A., Berger, J., 1983; J. Gen. Appl., Microbiol., 29, 41-50) erfolgt, welche eine maximale Zelldichte von 3*10³ Zellen/ ml und einer Generationszeit von 8-14 h in bakterienhaltigem (monoaxenischen) Medium liefert.

Im Sinne dieser Erfindung kann die erfindungsgemäße GLA Gewinnung und Isolierung bzw. GLA-Herstellung aus der Biomasse von Colpidium unmittelbar erfolgen oder über ein aus der Biomasse erhältliches Öl - insbesondere Lipidfraktion - erhalten werden, welches wiederum zuläßt GLA in reiner Form zu erhalten (nachstehend erfindungsgemäße GLA). Die GLA wird direkt oder aus einem Lipid (wie Glykolipide oder Phospholipide u.a.) mittels saurer Katalyse oder Hydrolyse unter Esterspaltung gewonnen.

Daher ist ebenfalls ein Gegenstand der Erfindung die Biomasse und / oder Öl als solche enthaltende Lipide.

Unter Biomasse wird zum einen Vorkommen an Protozoen aus der Gattung Colpidium als solche verstanden, als auch erfindungsgemäß fermentierte und kultivierte (siehe Beispiel) behandelte Protozoen der Gattung Colpidium. Sowohl Biomasse, Öl, insbesondere Lipidfraktionen, wie die daraus gereinigte GLA in reiner Form können als Grund- und Zusatz- oder Hilfsstoff für Nahrungsmittel, Pharmazeutika oder Kosmetika verwendet werden.

Daher betrifft die Erfindung ebenfalls ein Arzneimittel enthaltend das erfindungsgemäße GLA aus obiger Biomasse und/oder Öl und/oder in reiner Form und gegebenfalls pharmazeutisch annehmbare Zusatz- und/oder Hilfsstoffe.

Weiterhin betrifft die Erfindung ein Verfahren zur Herstellung eines Arzneimittels zur humanen und/oder veterinären Behandlung von Herz-Kreislauferkrankungen, Diabetes, Krebs und Arthritis dadurch gekennzeichnet, daß das erfindungsgemäße GLA mit pharmazeutisch annehmbaren Zusatz- und/oder Hilfsstoffen formuliert wird. Weiterhin betrifft die Erfindung ein Lebensmittelzusatzstoff in Tier- und Menschennahrung enthaltend die erfindungsgemäße GLA, vorzugsweise in reinster Form für die Säuglingsnahrung.

Ebenfalls betrifft die Erfindung einen kosmetischen Grund- und/oder Zusatzstoff enthaltend die erfindungsgemäße GLA.

Als bevorzugtes Verfahren zur Gewinnung von Öl und /oder GLA in reinster Form ist das Supercritical fluide reaction (SFR) Verfahren besonders geeignet. In einer besonderen Ausführungsform erfolgt die Herstellung von GLA mittels der kombinierten SFR/SFE (supercritical fluide extraction) Technologie, wie in der deutschen Patentanmeldung 198 32 784.6 beschrieben, auf die sich hier ausdrücklich bezogen wird. Es sind aber prinzipiell ebenfalls andere chromatographische Verfahren, die dem Fachmann bekannt sind, nicht ausgeschlossen.

Ein weiterer Gegenstand der Erfindung betrifft die Fermentation und Kultivierung von Colpidium zur Gewinnung und Isolierung der erfindungsgemäßen GLA in einem komplexen (d.h. kein Mangelmedium) und axenischen (d.h. ohne Nahrungsorganismen) Medium, welches bis zu 1-5%, besonders bevorzugt 0,5 - 2,5 % und ganz besonders bevorzugt 2% Magermilchpulver enthält, neben einer Vitamin- (z.B. Hefeextrakt) und Kohlenhydratquelle (siehe Beispiel) und weitere Hilf- und Zusatzstoffe.
Die nachfolgenden Beispiele dienen zur näheren Erläuterungen der Erfindung, ohne dieselbe auf in den Beispielen beschriebenen Produkte und Ausführungsformen einzuschränken.

### Beispiele:

### a.) Kultivierung:

### Magermilchmedium:

- 2 % (w/v): Magermilchpulver (Fa. Oxoid, Unipath, Wesel)
- 0,5 % (w/v): Hefeextrakt (Fa. Difco, Detroit, USA)
- 1 % (w/v): Glucose (getrennt autoklaviert)
- 0,1 % (v/v): Eisenspurlösung
- in: H₂O (bidest.)

### Eisenspurlösung:

- 34 mM: tri-Natriumcitrat
- 90 mM: FeCl₃
- in: H₂O (bidest.)

### Langzeitkultivierung:

Im Dunkeln bei 25°C in einem Reagenzglas mit 12 ml H₂O bidest und einer Kichererbse, autoklaviert; alle drei Monate Überführung von 200 µl dieser Kultur in ein neues Röhrchen.

### Vorkultur:

Schütteln (60 rpm, Schüttelbank Fa. Infors, Bottmingen) von 10 ml MM in einem 100-ml-Erlenmeyerkolben bei 25°C, im Dunkeln; nach 3,5 d (spät log. Wachstumsphase) können mit 200 µl dieser Kultur 10 ml neu angeimpft werden.

### Schüttelkultur:

Schütteln (100 rpm, Infors HT, Fa. Infors, Bottmingen) von 100 ml MM in einem 500-ml-Erlenmeyerkolben bei 25°C, im Dunkeln; nach 3,5 d Erreichen der spätlogarithmischen Wachstumsphase.

### b.) Fermentation:

Die Fermentation von Colpidium mit dem Bioreaktorsystem Biostat Q, (B. Braun Biotech international, Melsungen) erfolgt unter diesen Bedingungen:
- pH-Wert konstant bei 7,0
- Temperatur konstant bei 22 - 31 °C
- p(O₂) > 20% Luftsäftigung
- Drehzahl des Magnetrührers:
   100 mm bei 0,33 l Volumen, bzw.
   max. 280 mm bei 0,66 l-Volumen (Scheiben- bzw. Propellerrührer), abhängig vom p(O₂)

Nach In situ-Sterilisation des Fermenters und Zugabe der Glucose-Lösung wird mit einer Anfangszelldichte von 1*10⁵ Zellen/ ml aus Schüttelkulturen der spätlogarithmischen Wachstumsphase inokuliert.

Bei Schaumbildung während der Fermentation wird bis max. 0,03% Silikonöl zugegeben.

### c.) Analytik:

Der laufenden Fermentation werden regelmäßig Aliqots entnommen, mit Toluol/ Ethanol extrahiert und das Extrakt in Dichlormethan/ Methanol gelöst. Die Proben werden mit Trimethylsulfoniumhydroxid (TMSH) umgeestert (50 - 100 µl TMSH /2 mg Öl). Bevorzugt erfolgt eine direkte Umesterung aus der lyophilisierten Biotrockenmasse (BTM).

Die erhaltenen Fettsäure-Methylester werden Gas-chromatographisch analysiert mit dem Gaschromatograph HP 6890 Series, HP Automatic-Liquid-Sampler G1512A mit Flammenionisationsdetektor (HP FID) und einer polaren Kapillarsäule mit chemisch gebundenem PEG-2-Nitrophtalsäureester (Permabond FFAP, Länge 25m, Durchmesser 250µm, Filmdicke 0,1µm, Macherey-Nagel GmbH, Düren, Germany). Die Identifizierung und Quantifizierung der Fettsäuren erfolgt durch Vergleich der erhaltenen Retentionszeiten mit denen bekannter Fettsäuremethylester, und zwar computergestützt durch die HP Chem-Station (Hewlett-Packard-Company, Wilmington, USA).
Im Vergleich zur industriell betriebenen Gewinnung von GLA aus pflanzlichen Ölen weist die GLA-Produktion mit Ciliaten aufgrund der kurzen Generationszeiten und höheren absoluten GLA-Gehalte grundsätzlich eine wesentlich höhere Raum-Zeit-Ausbeute auf.

Ein Vergleich der von Colpidium campylum erzielten Ausbeute von 300 mg GLA/ l Kultur mit etablierten Verfahren zur GLA-Gewinnung aus Mikroorganismen belegt dessen hohes Potential als GLA-Produzent. Aus Schüttelkulturen der Mikroalge Spirulina platensis ließen sich bisher nur 38 mg GLA/ l Kultur gewinnen (Mahajan G., Kamat, M., 1995; Appl. Microbiol. Biotechnol., 43, 466-9), Kulturen der Pilze Mucor rouxii (Lindberg, A.M., Hansson, L., 1991; Appl. Microbiol. Biotechnol., 36, 26-8) und Rhizopus arrhizus (Kristofikova, L., Rosenberg, M., Vlnova, A., Sajbidor, J., Certik, M., 1991; Folia Microbiol., 36, 451-5) erreichten Ausbeuten von bis zu 330 bzw. 400 mg GLA/ l Kultur. Fermentation des Ciliaten Tetrahymena rostrata (Gosselin, Y., Lognay, G., Thonart, P., 1989; Biotechnol. Lett., 11 (6), 423-6) ergab eine maximale Ausbeute von 500 mg GLA/ l Kultur, eine Massenkultivierung von Tetrahymena thermophila (Kiy, T., 1993; Dissertation, Universität Münster) in Magermilchmedium erzielte 1,1 g GLA/ l Kultur. Allerdings ist die Lipid-Zusammensetzung aus letzteren Organismen komplexer als aus Colpidium, wodurch die präparative Gewinnung von GLA aus Tetrahymena erschwert wird.

### Beschreibung der Figuren:

Fig. 1 zeigt das Fettsäure-Spektrum von Tetrahymena thermophila. Gaschromatogramm des Fettsäure-Spektrums von Tetrahymena thermophila erhalten nach der im Abschnitt "Analytik" beschriebenen Methode. GLA eluiert nach 18,2 min. Sie hat einen Anteil von 16,3% (w/w) am gesamten Fettsäuregehalt.
Fig. 2 zeigt den phylogentischen Stammbaum verschiedener Ciliaten-Arten nach: Coyne, R.S., Yao, M.-C., 1996; Genetics, 144: 14779-87

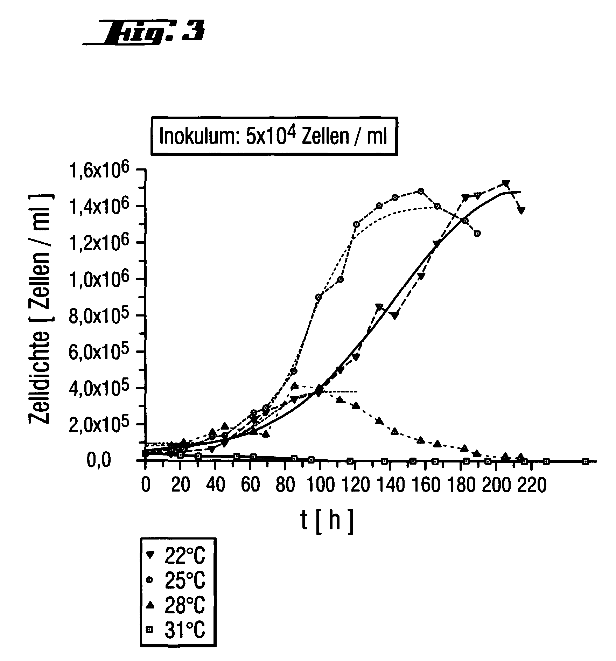

Fig. 3 zeigt die Fermentation von C. campylum bei 22°C, 25°C, 28°C und 31°C; Inokulum jeweils 5*10⁴ Zellen/ ml. Die kürzeste Generationszeit erhält man bei 25 °C (20,8 h) mit einer maximalen Zelldichte von 1,4 * 10 ⁶ ml⁻¹. Bei 22 °C, hingegen, erhält man eine Zelldichte von 1,5 * 10 ⁶ ml⁻¹ bei einer Generationszeit von 45,4 h.

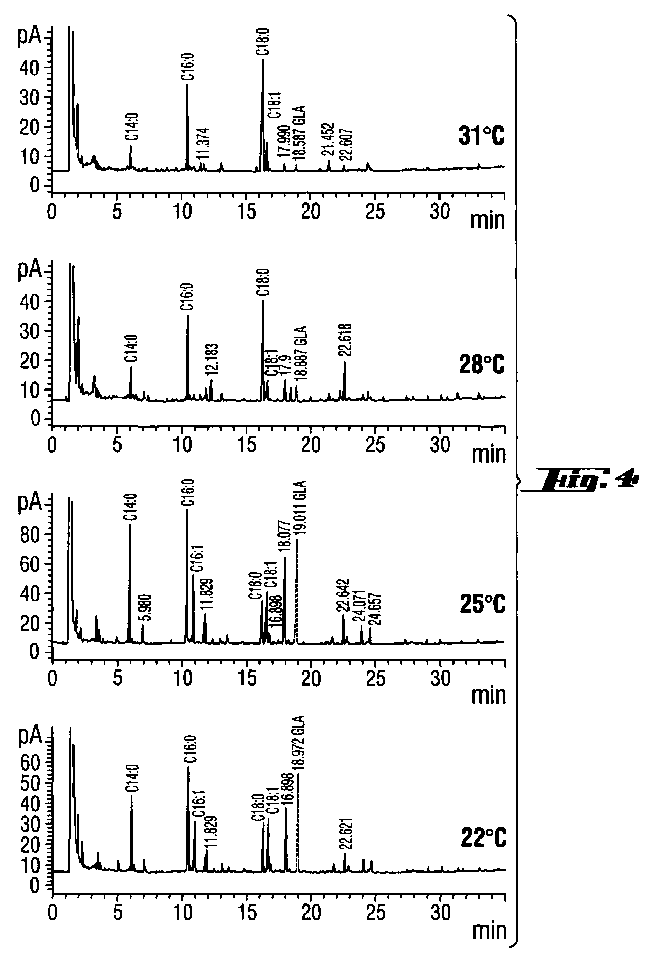

Fig. 4 zeigt das Gas-Chromatogramm nach 85 h Fermentation bei 22 - 31 °C (vgl. entsprechend Fig. 3). GLA - Gehalt beträgt 19% am Gesamt-Lipid; dies sind 5 mg GLA pro g Biotrockenmasse (BTM), bzw. 50 mg GLA pro l Kulturmedium.

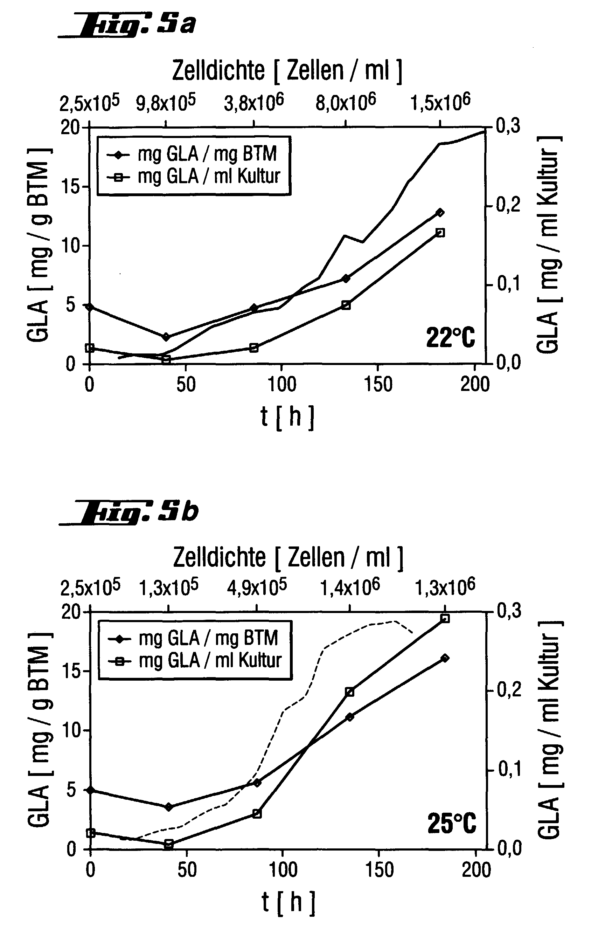

In Fig. 5A und B ist gezeigt, wie sich die GLA-Produktion im Verlauf der Fermentation bei 22 und 25 °C entwickelt: (B) Maximaler absoluter GLA-Gehalt stellt sich in der früh-stationären Wachstumsphase bei einer Zelldichte von 1,4 * 10 ⁶ ml⁻¹ ein.

Bei der Fermentation unter optimalen Wachstumsbedingungen (25°C) verschiebt sich die relative Fettsäure-Zusammensetzung der C. campylum-Kulturen von einem hohen Anteil an gesättigten Fettsäuren hin zu einem hohen GLA-Gehalt. Während der Lag-Phase liegen 15% GLA vor. Als maximalen relativen GLA-Wert erhält man in der logarithmischen Phase nach ca. 160 h 19% GLA; bei Fermentation bei 22°C sogar 25%; hier allerdings aufgrund des langsameren Wachstums und ca. 50 h später als bei 25 °C. In der weiteren Kultur nimmt der relative GLA-Gehalt wieder ab.

Die höchsten quantitativen GLA-Ausbeuten werden bei der Kultivierungstemperatur von 25°C erzielt, nämlich nach 185 h Fermentation, in der Stationärphase: 13 mg GLA/ g BTM und 290 mg GLA/ l Kultur. Dem stehen bei einer Temperatur von 22°C maximal 7 mg GLA/ g BTM und 170 mg GLA/ l Kultur gegenüber, und zwar nach einer Fermentationsdauer von 220 h.

Die höchsten relativen GLA-Gehalte von C. campylum werden in der Phase logarithmischen Wachstums erzielt, die höchsten absoluten GLA-Gehalte, hingegen, nach dem Übergang von der Wachstumsphase in die Stationärphase. Dies wird - neben Erreichen der maximalen Zelldichte - durch eine Zunahme des Gesamtfettgehaltes in der Stationär-Phase erklärt (Erwin, J., Bloch, K., 1963; J. Biol. Chem., 238 (5), 161 8-24; Holz, G.G., Conner, R., 1973; Biology of Tetrahymena; Stroudsburg, PA).

## Patentansprüche

1. Verwendung der Gattung Colpidium als biologische Quelle zur Gewinnung und Isolierung von γ- Linolensäure.

2. Verfahren zur Herstellung von γ- Linolensäure nach Anspruch 1, dadurch gekennzeichnet, daß Colpidium kultiviert und fermentiert wird.

3. Verfahren nach Anspruch 2, dadurch gekennzeichnet, daß die Fermentation und Kultivierung bei 19-28° C erfolgt.

4. Verfahren zur Herstellung von γ- Linolensäure nach Anspruch 1 bis 3, dadurch gekennzeichnet, daß γ- Linolensäure aus der Biomasse und/oder Öl und/oder Lipid isoliert wird.

5. Biomasse und/oder Öl enthaltend γ- Linolensäure aus Colpidium.

6. Komplexes und axenisches Medium zur Kultivierung und Fermentation nach Anspruch 2 und 3 enthaltend vorzugsweise 2% Magermilchpulver, Vitamin- und Kohlenstoffquelle und weitere Hilf- und Zusatzstoffe.

7. Arzneimittel enthaltend γ- Linolensäure gemäß einem der Ansprüche 1 ― 5 und gegebenenfalls pharmazeutisch annehmbare Zusatz- und/oder Hilfsstoffe.

8. Verfahren zur Herstellung eines Arzneimittels zur humanen und/oder veterinären Behandlung von Herz-Kreislauferkrankungen, Diabetes, Krebs und Arthritis dadurch gekennzeichnet, daß γ- Linolensäure gemäß Ansprüche 1-5 mit pharmazeutisch annehmbaren Zusatz- und/oder Hilfsstoffen formuliert wird.

9. Verwendung der γ- Linolensäure gemäß einem der Ansprüche 1 - 5 als Lebensmittelzusatzstoff in Tier- oder Menschennahrung.

10. Verwendung der γ- Linolensäure gemäß Anspruch 1- 5 als kosmetischer Grund- und Zusatzstoff.
